# EUROPÄISCHE PATENTSCHRIFT

(11) **EP 3 863 552 B1**
(45) Veröffentlichungstag und Bekanntmachung des Hinweises auf die Patenterteilung: **17.06.2026**
(21) Anmeldenummer: 19789618.6
(22) Anmeldetag: 10.10.2019
(51) Int. Cl.: A61B 90/98, A61B 17/00

(54) **INTELLIGENTES HANDSTÜCK**
INTELLIGENT HANDPIECE
PIÈCE À MAIN INTELLIGENTE

(30) Priorität: 11.10.2018 DE 102018125181
(43) Veröffentlichungstag der Anmeldung: 18.08.2021
(73) Patentinhaber: Aesculap AG, 78532 Tuttlingen (DE)
(72) Erfinder: HÖGERLE, Roland-Alois, 78532 Tuttlingen (DE); KAHLER, Thomas-Erwin, 78606 Seitingen-Oberflacht (DE); MACHILL, Martin, 78604 Rietheim-Weilheim (DE)
(74) Vertreter: Winter, Brandl - Partnerschaft mbB
(86) Internationale Anmeldenummer: PCT/EP2019/077492
(87) Internationale Veröffentlichungsnummer: WO 2020/074648

(56) Entgegenhaltungen:
- DE-A1- 10 135 155
- DE-A1- 102008 024 438
- DE-A1- 102011 050 192
- US-A1- 2004 133 189

## Beschreibung

### Technisches Gebiet

Die vorliegende Erfindung betrifft eine Kommunikationsvorrichtung, insbesondere für ein chirurgisches Kupplungssystem bzw./oder eines chirurgischen Kupplungssystems, gemäß dem Oberbegriff des Anspruchs 1.

Wie aus der DE 10 225 857 A1 bereits bekannt, ist eine Steuereinheit mit mehreren Anwendungsteilen/Handstücken über ein chirurgisches Kupplungssystem verbindbar, welches später genauer beschrieben wird. In der Chirurgie kommen je nach Einsatzzweck verschiedene Motoreinheiten mit gleichen oder unterschiedlichen Motoren, insbesondere chirurgischen Motoren, zum Einsatz. Je nach erforderlicher Drehzahl werden entsprechende Motortypen ausgewählt und in ein Anwendungsteil integriert. Durch das in der DE 10 225 857 A1 beschriebene Kupplungssystem ist nun mehr nur noch eine Steuereinheit für mindestens zwei Anwendungsteile/Handstücke erforderlich. Somit kann im Operationsbereich durch die Verringerung der Anzahl an Steuergeräten die Übersichtlichkeit erhöht und die Wahrscheinlichkeit einer Fehlbedienung der Steuereinheiten verringert werden.

DE 101 35 155 A1 betrifft einen Instrumentensatz mit Identifikationsfunktion, der lösbar mit einem Hauptkörper einer medizinischen Vorrichtung zur Verwendung bei Diagnose und Behandlung verbunden ist.

Aus der DE 10 2011 050 192 A1 ist eine elektrische Schalteinrichtung bekannt, die auf dem vorstehend beschriebenen Stand der Technik aufbaut und das chirurgische Kupplungssystem näher beschreibt. Das chirurgische Kupplungssystem besteht aus einer ersten Kupplungsvorrichtung und einer zweiten Kupplungsvorrichtung, welche als eine (Steck-)Verbindung ausgebildet sind, um ein Anwendungsteil/Handstück mit einer/einem Steuereinheit/Steuergerät zu verbinden. Die erste Kupplungsvorrichtung befindet sich an einem Ende eines Versorgungskabels, dessen anderes Ende mit der Steuereinheit verbunden ist und diese mit dem Anwendungsteil verbindet. Die zweite Kupplungsvorrichtung ist Teil des Anwendungsteils, in welchem der Elektromotor integriert ist. Hierbei bestehen die erste Kopplungsvorrichtung und die zweite Kupplungsvorrichtung jeweils aus mindestens zwei mechanisch miteinander in Eingriff bringbaren elektrischen Kupplungskontakten.

Der integrierte Elektromotor weist drei Motorwicklungen auf, welche in einer Sternschaltung zueinander verschaltet sind. Jede Motorwicklung ist über jeweils eine Leitung mit der zweiten Kupplungsvorrichtung verbunden. Das heißt, in Bezug auf ein Beispiel, in welchem jede Kupplungsvorrichtung mit jeweils vier elektrischen Kupplungskontakten ausgebildet ist, sind zwei elektrische Kupplungskontakte der zweiten Kupplungsvorrichtung mit jeweils einer Leitung verbunden. Die zwei anderen elektrischen Kupplungskontakte der zweiten Kupplungsvorrichtung sind parallel geschalten und mit der dritten Leitung verbunden. Vor einem der beiden parallel geschalteten elektrischen Kupplungskontakte ist ein Widerstandselement zwischengeschaltet, welches ein Kodierelement bildet zum Kodieren der Art des Handstücks. Über diesen Signalpfad kann mittels einer Widerstandsmessung über das Widerstandselement die Art des in das Anwendungsteil integrierten Elektromotors bestimmt werden und diese Information über den Signalpfad an die Steuereinheit weitergegeben werden, um den Motor dieser Information angepasst anzutreiben.

Hierbei definiert das Kupplungssystem drei unterschiedliche Stellungen/Zustände. Die erste und die zweite Kupplungsvorrichtung befinden sich demnach in einer Trennstellung, wenn diese beiden vollständig voneinander getrennt sind. Das heißt, dass keiner der elektrischen Kupplungskontakte von der ersten Kupplungsvorrichtung in Kontakt stehen mit den elektrischen Kupplungskontakten der zweiten Kupplungsvorrichtung und auch nicht mechanisch miteinander in Eingriff stehen. In einer AUS-Stellung steht mindestens ein erster elektrischer Kupplungskontakt der ersten Kupplungsvorrichtung mit mindestens einem ersten elektrischen Kupplungskontakt der zweiten Kupplungsvorrichtung außer Eingriff. Das heißt, wenn die erste und die zweite Kupplungsvorrichtung jeweils mit vier elektrischen Kupplungskontakten ausgebildet sind, und zwei elektrische Kupplungskontakte der ersten Kupplungsvorrichtung mit zwei elektrischen Kupplungskontakten der zweiten Kupplungsvorrichtung miteinander in Eingriff stehen, befindet sich das Kupplungssystem in einer AUS-Stellung. In einer AN-Stellung befindet sich die Kupplungsvorrichtung, wenn alle elektrischen Kupplungskontakte der ersten Kupplungsvorrichtung mit allen entsprechenden elektrischen Kupplungskontakten der zweiten Kupplungsvorrichtung elektrisch leitend miteinander in Kontakt stehen.

Somit kann das Kupplungssystem als Schalteinrichtung verwendet werden und weist eine Doppelfunktion auf. Zum einen kann es eine mechanische Verbindung zwischen dem Handstück und beispielsweise der Versorgungsleitung herstellen. Zum anderen kann es auch als Schalteinrichtung genutzt werden, um beispielsweise Motorwicklungen eines Elektromotors des Handstücks bei Bedarf mit Strom versorgen zu können.

Des Weiteren kann dadurch die zusätzliche Anforderung gestellt werden, in welcher die Art eines Handstücks automatisch von einer Steuer- und/oder Regelungseinrichtung eines Antriebssystems abgefragt werden kann. Diese Kommunikation kann allerdings nur in der vorstehend beschriebenen AUS-Stellung stattfinden und der Signalpfad muss über diverse Litzen/Leitungen, bzw. der Motorwicklung und mehrere Schnittstellen, wie Steckkontakte, hinweg an die Steuereinheit geleitet werden. Dies hat den Hintergrund, dass in der AN-Stellung, wenn beide der parallel geschalteten elektrischen Kupplungskontakte in Kontakt stehen, der Signalpfad über das Widerstandselement kurzgeschlossen ist und somit kein Informationsaustausch zwischen dem Anwendungsteil und der Steuereinheit mehr möglich ist. Der Weg des Signalpfads ist des Weiteren nachteilig, da es zu Verzerrungen, Fehlübertragungen und zeitlichen Verzögerungen, somit in Summe zu Toleranzen kommen kann, welche dann an der Steuereinheit als falsches Signal gewertet werden können. Um einen solchen Kommunikationspfad in der AN-Stellung aufrechtzuerhalten und somit einen Informationsaustausch zwischen dem Anwendungsteil und der Steuereinheit zu ermöglichen, lässt sich durch eine Erhöhung der Anzahl der Versorgungsleitungen/-litzen für den Elektromotor lösen.

Der benachbarte Stand der Technik ist auch aus den Druckschriften US 2004/133189 A1 und DE 10 2008 024438 A1 zu entnehmen.

Der Erfindung liegt daher als eine Aufgabe zugrunde, eine Kommunikationsvorrichtung für ein chirurgisches Kupplungssystem gemäß der vorstehenden Beschreibung zur Verfügung zu stellen, die eine ständige, zuverlässige und sichere Kommunikation zwischen dem Anwendungsteil und der Steuereinheit ohne der Verwendung von weiteren Leitungen ermöglicht und dies in einem einfachen System realisiert.

Diese Aufgabe wird erfindungsgemäß durch eine Kommunikationsvorrichtung mit den Merkmalen des Anspruchs 1 gelöst. Vorteilhafte Weiterbildungen der Erfindung sind Gegenstand der beigefügten Unteransprüche. Die Erfindung ist im beigefügten Anspruchssatz beschrieben.

Der Erfindung liegt die allgemeine Idee zugrunde, für ein chirurgisches Kupplungssystem, welches mindestens ein Anwendungsteil/Handstück und eine Steuereinheit miteinander verbindet, eine ständige bzw. über einen längeren Zeitraum anhaltende/stattfindende Kommunikation/Verbindung bereitzustellen. In dem Anwendungsteil ist ein Elektromotor zum Antreiben eines an das Anwendungsteil angebrachten/eingesteckten chirurgischen Werkzeugs untergebracht. Das Anwendungsteil kann beispielsweise auch als ein Interface zum Anschließen an einen Roboter ausgebildet sein.

Gemäß der zugrundeliegenden allgemeinen Idee besteht zwischen dem Anwendungsteil und der Steuereinheit eine Kommunikationsverbindung, welche sowohl im AUS-Zustand als auch im AN-Zustand einen Informationsaustausch vorsieht, um aus den in der Steuereinheit gesammelten Daten sämtliche Informationen aller verwendeten individuellen Anwendungsteile in der Steuereinheit auszulesen. Diese Informationen können eine Auswertung der Grundinformationen, wie die Anzahl der Nutzungen, bspw. im OP, die Gesamtlaufzeit, die Laufzeit pro Nutzung, sowie die Laufzeit im Rechts-/Linkslauf, Start/Stopp-Zyklen, die Stromaufnahme etc. des jeweiligen Anwendungsteils sein. Darüber hinaus geht die in der Steuereinheit durchgeführte Auswertung der Sensorsignale über die Häufigkeit des verwendeten Werkzeugtyps, Feuchtezustände, Temperaturen, Anzahl der Aufbereitungen, etc. bis hin zu einer Statistik über alle genutzten Anwendungsteile.

Für die Aufrechterhaltung der Kommunikationsverbindung zwischen dem Anwendungsteil und der Steuereinheit zur Übertragung diverser Informationen und Sensorsignale des Anwendungsteils, sind keine weiteren Litzen/Leitungen, im Versorgungs-/Anschlusskabel vorgesehen. Die Anzahl der Leitungen wird hierbei über die Anzahl der Motorwicklungen, vorzugsweise drei Motorwicklungen, des Elektromotors festgelegt.

Die Verwendung einer in das Anwendungsteil integrierten Kommunikationsvorrichtung bietet eine zuverlässige und sicherere Informationsübertragung, welche sowohl in einem AUS- als auch einem AN-Zustand, gemäß vorstehender Definition aus dem Stand der Technik, verwendbar ist. Diese/r Kommunikationsverbindung/Signalweg kann somit über einen längeren Zeitraum oder auch ständig aufrechterhalten werden.

Im Einzelnen hat die Kommunikationsvorrichtung für ein chirurgisches (elektrisches) Kupplungssystem eine in das Anwendungsteil integrierte intelligente Vorrichtung, die dazu ausgebildet ist, eine Kommunikation zwischen dem Anwendungsteil und der Steuereinheit in dem AUS-Zustand und/oder in dem AN-Zustand aufrechtzuerhalten. Ein Versorgungskabel zwischen dem Anwendungsteil und der Steuereinheit hat maximal drei Leitungen, entsprechend der Anzahl an Motorwicklungen des Elektromotors. Unter einer intelligenten Vorrichtung wird allgemeint eine Vorrichtung verstanden, welche sich mit der Automatisierung intelligenten Verhaltens und dem Maschinenlernen befasst. Die "Intelligenz" /intelligente Vorrichtung der vorliegenden Erfindung hat diverse Grundinformationen des Anwendungsteils, wie bspw. Seriennummer, Herstelldatum, Instandhaltungsdaten, etc. Diese Grundinformationen gibt die intelligente Vorrichtung an die Steuereinheit weiter. In der Steuereinheit werden die Informationen gesammelt/gespeichert und ausgewertet/verarbeitet. Durch die Ausgestaltung der Kommunikationsvorrichtung kann die Anzahl der erforderlichen elektrischen Kontaktelemente minimiert werden, insbesondere auf die Anzahl der vorhandenen Motorwicklungen des Elektromotors. Auf weitere Leitungen, bspw. um bei herkömmlichen Antriebssystemen den Elektromotor zu betätigen und gegebenenfalls dessen Typ oder Bauart abzufragen, kann in der vorliegenden Erfindung verzichtet werden. Zum Steuern und/oder Regeln des Elektromotors ist es ferner vorteilhaft, wenn die Steuereinheit insbesondere mit dem chirurgischen Handstück/Anwendungsteil derart zusammenwirkend ausgebildet ist, dass sie die Art des Anwendungsteils automatisch abfragen und erkennen kann.

Bevorzugt ist, wenn die intelligente Vorrichtung direkt in einen Signalweg zwischen einem der parallel geschalteten elektrischen Kupplungskontakte der zweiten Kupplungsvorrichtung und einer entsprechenden Motorwicklung des Elektromotors geschalten ist. Somit kann die intelligente Vorrichtung in der AUS-Stellung ständig mit Energie versorgt werden und mit der Steuereinheit kommunizieren.

Die Kommunikation zwischen dem Anwendungsteil und der Steuereinheit erfolgt kabellos. Auf diese Weise ist eine Kommunikation in der AN-Stellung trotz kurzgeschlossenem Signalpfad, gemäß vorstehender Beschreibung aus dem Stand der Technik, möglich. Solche kabellose Übertragungsverfahren sind Datenübertragungsverfahren, die den freien Raum als Übertragungsmedium nutzen und auf ein Kabel in Form eines elektrischen Leiters oder eines Lichtwellenleiters verzichten. Vorzugsweise werden Übertragungsverfahren wie Bluetooth oder WLAN verwendet. Alternativ können Übertragungsverfahren wie ZigBee, NFC, Wibree oder andere, je nach Datenmenge und benötigter Reichweite, verwendet werden.

In dem Anwendungsteil ist ein Energiespeicher vorgesehen oder an diesem angebracht, der mit der intelligenten Vorrichtung elektrisch verbunden ist. In dem AUS-Zustand kann der zusätzliche Energiespeicher über die Versorgungsleitung aufgeladen werden und die intelligente Vorrichtung kann ständig, bzw. in dem AN-Zustand über einen längeren Zeitraum hinweg kabellos mit der Steuereinheit kommunizieren. Auch ein komplett abgenommenes Anwendungsteil bzw. sich im Trennzustand befindendes Anwendungsteil, ist in der Lage weiterhin mit der Steuereinheit zu kommunizieren bis der Energiespeicher erschöpft ist. Als ein Energiespeicher können zumindest ein Akku verwendet werden, bspw. Lithium-Ionen bzw. Lithium-Polymer Akkus. Vorteilhaft ist hierbei die Verwendung von kleinen und leichten Energiespeichern.

Es ist vorteilhaft, wenn die intelligente Vorrichtung über eine Entladungsschutzsicherung verfügt. Durch Tiefentladung eines Energiespeichers kann es je nach Energiespeichertyp zu unterschiedlichen Schädigungen kommen. Somit ist es vorteilhaft, wenn die Entladungsschutzsicherung dafür sorgt, dass die Spannung nicht unter eine Entladeschlussspannung absinkt. Die vorgesehene Entladungsschutzsicherung in der intelligenten Vorrichtung hat daher die Aufgabe, dass dies in dem AN-Zustand und Trennzustand verhindert wird. Hierbei sorgt die Entladungsschutzsicherung für eine elektrische Trennung des Energiespeichers von der intelligenten Vorrichtung, wenn je nach Energiespeichertyp eine festgesetzte Spannung, bis zu welcher der Energiespeicher entladen darf, erreicht ist.

Vorzugsweise weist die intelligente Vorrichtung mehrere Eingänge für unterschiedliche Sensorsignale auf. Hierbei sind Sensoren, insbesondere TemperaturSensoren, Sensoren ob ein Werkzeug bzw. welcher Werkzeugtyp vorhanden ist, Feuchtigkeitssensor, Sensor zur Erkennung für Aufbereitungen, etc. aus dem Anwendungsteil, vorgesehen. Diese Sensoren liefern/senden somit mittels Sensorsignalen weitere Informationen an die Steuereinheit über einen Zeitraum, bis der Energiespeicher erschöpft ist, bzw. die festgesetzte Spannung erreicht hat, wie vorstehend beschrieben.

Gemäß einem weiteren Aspekt der Erfindung ist die intelligente Vorrichtung dazu ausgebildet, mit der Steuereinheit zu kommunizieren und/oder zwischen mehreren Anwendungsteilen untereinander zu kommunizieren. In anderen Worten bedeutet das, dass die intelligente Vorrichtung in der Lage ist, ständig mit der Steuereinheit und auch untereinander zwischen den anderen Anwendungsteilen zu kommunizieren und Informationen auszutauschen. Es könnte bspw. mittels dieser Kommunikation unter den Anwendungsteilen selbst, eine vorgeschrieben Reihenfolge bei dessen Verwendung während einer Operation sichergestellt werden. Somit können fehleranfällige Abläufe von Operationen unterstützt und die Verfahrenssicherheit erhöht werden. Eine solche Kommunikation zwischen den Anwendungsteilen kann des Weiteren der Fehlervermeidung, der Erhöhung der Patientensicherheit und der Entlastung des Chirurgen dienen. Zudem ist es vorteilhaft, wenn eine solche Kommunikation in Echtzeit erfolgt.

Alternativ kann die Spannungsversorgung der intelligenten Vorrichtung indirekt, insbesondere induktiv erfolgen. Aufgrund der im Anwendungsteil gegebenen Nähe der intelligenten Vorrichtung zu dem Energiespeicher, bietet die induktive Kopplung eine Alternative zur direkten Spannungsversorgung. Unter einer induktiven Kopplung wird die gegenseitige magnetische Beeinflussung zweier oder mehrerer räumlich benachbarter elektrischer Stromkreise durch die elektromagnetische Induktion infolge einer Änderung des magnetischen Flusses verstanden. Hierbei bewirkt eine stromdurchsetzte (erste) Leiterschleife die Erzeugung einer magnetischen Flussdicht in ihrer räumlichen Umgebung. Eine induktive Energieübertragung weist im Nahbereich einen vergleichsweise hohen Wirkungsgrad auf. Dabei ist es von Vorteil, wenn der Abstand zwischen einem Sender, hier der Energiespeicher und einem Empfänger, hier die intelligente Vorrichtung, möglichst gering gehalten wird. Alternativ wäre eine weitere Möglichkeit, den Energiespeicher mittels induktiver Kopplung kabellos zu laden.

Vorteilhafterweise ist die intelligente Vorrichtung im Zentrum einer Spule, welche im Signalweg positioniert ist und sowohl zur Umsetzung der induktiven Kopplung nötig ist, um eine optimale induktive Spannungsversorgung garantieren zu können.

Gemäß einem anderen Aspekt der Erfindung ist die intelligente Vorrichtung mittels Harvesting aus den Leitungen der Spannungsversorgung des Elektromotors oder streuenden Magnetfeldern des Elektromotors mit Energie versorgt. Unter Harvesting ist zu verstehen, dass Energie aus der Umgebung gewonnen wird. Es kommen als Quellen bspw. Temperaturunterschiede, Bewegung (z.B. das Drücken eines Schalters, sich bewegende Maschinenteile), Licht (z.B. Umgebungslicht) in Frage, welche mit einem entsprechenden Energiewandler mit verschiedenen Leistungsparametern zur Energiegewinnung beitragen.

### Kurzbeschreibung der Figuren

Fig. 1 ist eine schematische Schaltskizze zur Veranschaulichung des Kupplungssystems gemäß einem Trennzustand der vorliegenden Offenbarung;
Fig. 2 ist eine schematische Schaltskizze zur Veranschaulichung des Kupplungssystems gemäß einem AUS-Zustand der vorliegenden Offenbarung;
Fig. 3 ist eine schematische Schaltskizze zur Veranschaulichung des Kupplungssystems gemäß einem AN-Zustand der vorliegenden Offenbarung;
Fig. 4 ist eine ausschnittsweise schematische Darstellung des Anwendungsteils gemäß einem ersten Ausführungsbeispiel;
Fig. 5 ist eine ausschnittsweise schematische Darstellung des Anwendungsteils gemäß einem zweiten Ausführungsbeispiel;
Fig. 6 ist eine ausschnittsweise schematische Darstellung des Anwendungsteils gemäß einem dritten Ausführungsbeispiel; und
Fig. 7 ist eine ausschnittsweise schematische Darstellung mehrerer Anwendungsteile.

### Beschreibung der Ausführungsbeispiele

Nachstehend werden Ausführungsbeispiele der vorliegenden Offenbarung auf der Basis der zugehörigen Figuren beschrieben. Gleiche oder funktional äquivalente Merkmale sind in den einzelnen Figuren mit denselben Bezugszeichen versehen und werden zweckmäßig nicht mehrfach beschrieben.

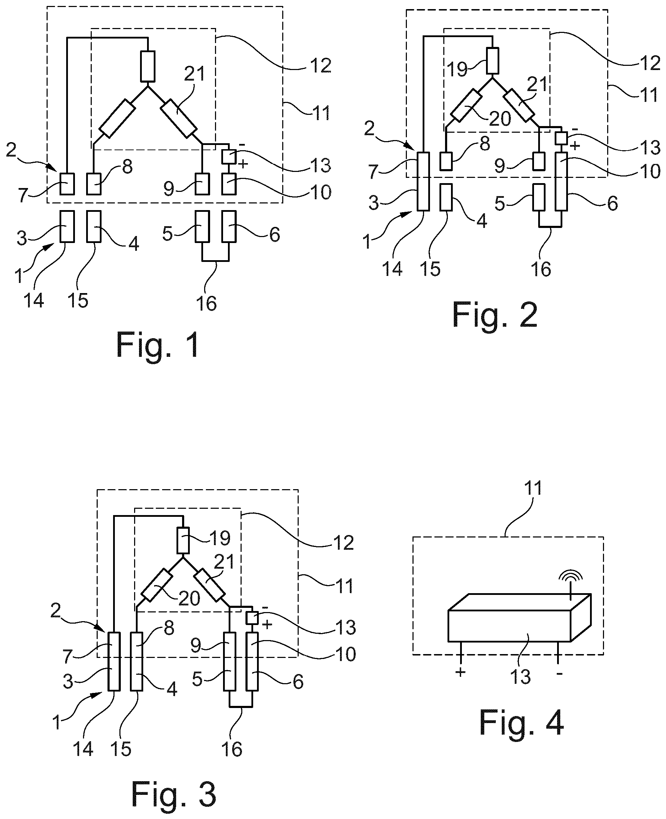

Fig. 1 ist eine schematische Schaltskizze zur Veranschaulichung eines Kupplungssystems gemäß einem Trennzustand. Die erste bzw. männliche chirurgische Kupplungsvorrichtung 1 und die zweite bzw. weibliche chirurgische Kupplungsvorrichtung 2 sind vollständig voneinander getrennt, stehen also außer Eingriff. Jede der beiden Kupplungsvorrichtungen 1 und 2 weist jeweils vier elektrische Kupplungskontakte auf. Die erste Kupplungsvorrichtung 1 hat die elektrischen Kupplungskontakte 3, 4, 5 und 6 und die zweite Kupplungsvorrichtung 2 hat die elektrischen Kupplungskontakte 7, 8, 9 und 10.

Ein Anwendungsteil/Handstück 11 ist als schematische Schaltskizze dargestellt. In dem Anwendungsteil 11 sind ein Elektromotor 12 und eine intelligente Vorrichtung 13 untergebracht/integriert. Des Weiteren können sich die Anwendungsteile 11 nicht nur äußerlich, sondern auch im Innenaufbau unterscheiden. Dies bedeutet bspw., dass die in den Anwendungsteilen 11 verbauten Elektromotoren 12 von unterschiedlichen Typen sein können und sich bspw. in ihren Kenngrößen, wie Minimaldrehzahl, Maximaldrehzahl, Maximalstrom und Maximaldrehmoment unterscheiden.

Des Weiteren ist das Anwendungsteil 11 mit den elektrischen Kupplungskontakten 7, 8, 9 und 10 der zweiten Kupplungsvorrichtung 2 bestückt. Der Elektromotor 12 dient als Antriebssystem für ein an das Anwendungsteil 11 anbringbares und lösbar verbundenes chirurgisches Werkzeug. Maximal drei Leitungen 14, 15 und 16 bilden ein Versorgungskabel. Über die drei Leitungen 14, 15 und 16 werden die Motorwicklungen 19, 20 und 21 des Elektromotors 12 mit Strom versorgt.

Der Kupplungskontakt 7 ist mit der Motorwicklung 19 elektrisch leitend verbunden. Die Motorwicklung 19 ist sternförmig mit den Motorwicklungen 20 und 21 verschaltet. Die Motorwicklung 20 ist ferner elektrisch leitend mit dem elektrischen Kupplungskontakt 8 verbunden. Die Motorwicklung 21 ist einerseits elektrisch leitend mit dem Kupplungskontakt 9 verbunden und andererseits mit der intelligenten Vorrichtung 13, die in Serie mit dem elektrischen Kupplungskontakt 10 geschalten ist. Der elektrische Kupplungskontakt 9 ist zu der intelligenten Vorrichtung 13 und dem elektrischen Kupplungskontakt 10 parallel geschalten. Über die intelligente Vorrichtung 13 kann eine Art bzw. ein Typ des Anwendungsteils 11 eindeutig identifiziert werden. Durch eine entsprechende Ausgestaltung bzw. Programmierung der Steuereinheit kann diese Bestimmung/Identifizierung automatisch erfolgen.

Fig. 2 ist eine schematische Schaltskizze zur Veranschaulichung des Kupplungssystems gemäß einem AUS-Zustand. In dem AUS-Zustand des Kupplungssystems werden die elektrischen Kupplungskontakte 7 und 10 der zweiten Kupplungsvorrichtung mit den elektrischen Kupplungskontakten 3 und 6 der ersten Kupplungsvorrichtung elektrisch leitend miteinander verbunden. Dabei wird ein Stromkreis geschlossen, in welchem der Kupplungskontakt 7 in Serie mit der Motorwicklung 19, der Motorwicklung 20, der intelligenten Vorrichtung 13 sowie dem Kupplungskontakt 10 geschaltet ist. In dem AUS-Zustand kann durch Anlegen einer Spannung an die Leitungen 14 und 16 die intelligente Vorrichtung 13 mit Strom versorgt werden und ein Informationsaustausch zwischen der intelligenten Vorrichtung 13 und der Steuereinheit ist in diesem Zustand dauerhaft möglich.

Fig. 3 ist eine schematische Schaltskizze zur Veranschaulichung des Kupplungssystems gemäß einem AN-Zustand. In dem AN-Zustand stehen die alle elektrischen Kupplungskontakte 3, 4, 5 und 6 der ersten Kupplungsvorrichtung 1 mit den entsprechenden elektrischen Kupplungskontakte 7, 8, 9 und 10 in Eingriff. Die Motorwicklungen 19, 20 und 21 des Elektromotors 12 können in dem AN-Zustand mittels der Steuereinheit in an das Anwendungsteil angepasster Weise versorgt werden.

Die Steuereinheit ist in der Lage den Elektromotor 12 in gewünschter Weise anzusteuern, beispielsweise um ein chirurgisches Werkzeug, welches mit dem Anwendungsteil 11 lösbar verbunden ist, durch den Elektromotor 12 in Rotation zu versetzen.

Im Folgenden stellt das vorstehend beschriebene Kupplungssystem die Basis der folgenden alternativen Ausführungsbeispiele dar.

### Erstes Ausführungsbeispiel

Fig. 4 ist eine ausschnittsweise schematische Darstellung des Anwendungsteils 11 gemäß einem ersten Ausführungsbeispiel. Die in das Anwendungsteil 11 integrierte intelligente Vorrichtung 13, welche direkt in den Signalweg zwischen der Motorwicklung 21 und dem elektrischen Kupplungskontakt 10 in Serie geschaltet ist, ist in der Lage sowohl in dem AUS-Zustand gemäß Fig. 2 ständig, als auch in dem AN-Zustand gemäß Fig. 3 und/oder in dem Trennzustand gemäß Fig. 1 über einen längeren Zeitraum zu kommunizieren.

Fig. 4 zeigt die intelligente Vorrichtung 13 des Anwendungsteils 11 mit der Möglichkeit ein drahtloses/kabelloses Informationsübertragungsverfahren zu verwenden, wie beispielsweise Bluetooth, WLAN, etc. Über diese Verbindung sendet die intelligente Vorrichtung 13 Informationen an die Steuereinheit. Die Steuereinheit sammelt die empfangenen Informationen, verarbeitet und wertet diese aus, um den Informationen entsprechend angepasst, den Elektromotor 12 zu steuern/regeln.

### Zweites Ausführungsbeispiel

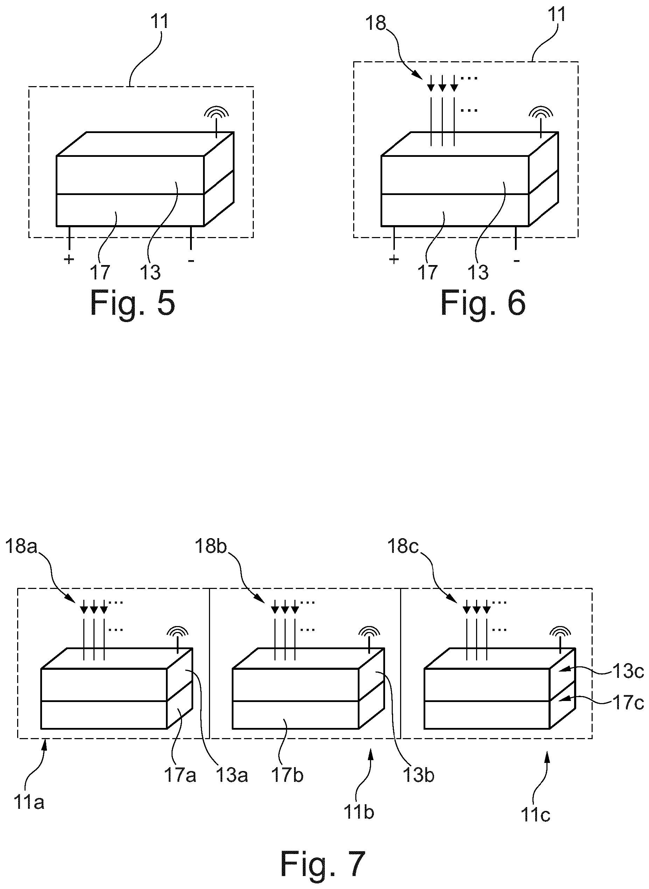

Fig. 5 zeigt eine ausschnittsweise schematische Darstellung des Anwendungsteils 11 gemäß einem zweiten Ausführungsbeispiel. Die intelligente Vorrichtung 13 ist mit einem Energiespeicher 17 verbunden und/oder an diesem angebracht. Der Energiespeicher 17 versorgt die intelligente Vorrichtung 13 mit Strom, sodass eine ständige bzw. in dem AN-Zustand über einen längeren Zeitraum hinweg kabellose Kommunikation mit der Steuereinheit möglich ist. Auch eine weiter anhaltende Kommunikationsverbindung zwischen einem komplett abgenommenen Anwendungsteil 11 und der Steuereinheit ist bis zur Erschöpfung des Energiespeichers 17 möglich.

Der Energiespeicher 17 kann in dem AUS-Zustand geladen werden, um dann im AN-Zustand und/oder der Trennstellung die intelligente Vorrichtung 13 mit Energie zu versorgen. Diese Energieübertragung kann gemäß dem einleitenden Teil, direkt oder indirekt erfolgen.

Bei der Verwendung zumindest eines Energiespeichers 17 verfügt die intelligente Vorrichtung 13 über eine Entladungsschutzsicherung (nicht dargestellt), die in dem AN-Zustand eine Tiefentladung und somit eine Schädigung des Energiespeichers 17 verhindert.

### Drittes Ausführungsbeispiel

Fig. 6 ist eine ausschnittsweise schematische Darstellung des Anwendungsteils 11 gemäß einem dritten Ausführungsbeispiel. Die intelligente Vorrichtung 13 ist gemäß dem zweiten Ausführungsbeispiel mit einem Energiespeicher 17 verbunden oder an diesen angebracht. Das Anwendungsteil 11 ist vorzugsweise mit Sensoren ausgestattet. Des Weiteren verfügt die intelligente Vorrichtung 13 über mehrere Signaleingänge 18 für diverse zusätzliche Sensorsignale, welche von der intelligenten Vorrichtung 13 an die Steuereinheit gesendet werden. Die Steuereinheit verarbeitet und wertet die empfangenen Sensorsignale aus, um den Elektromotor 12 den Signalen entsprechend angepasst zu steuern/anzutreiben.

Fig. 7 ist eine ausschnittsweise schematische Schaltskizze mehrerer Anwendungsteile. In Fig. 7 sind die Anwendungsteile 11a, 11b und 11c gezeigt, in welchem jeweils eine intelligente Vorrichtung 13a, 13b und 13c untergebracht ist. Jede intelligente Vorrichtung 13a, 13b und 13c ist mit einem Energiespeicher 17a, 17b und 17c verbunden oder an diesem angebracht. Die intelligente Vorrichtung 13a hat mehrere Signaleingänge 18a, die intelligente Vorrichtung 13b hat mehrere Signaleingänge 18b und die intelligente Vorrichtung 13c hat mehrere Signaleingänge 18c. Die entsprechenden Signaleingänge 18a, 18b und 18c geben die Signale der jeweiligen Sensoren der entsprechenden Anwendungsteile 11a, 11b und 11c an die entsprechende intelligente Vorrichtung 13a, 13b oder 13c weiter. Es versteht sich von selbst, dass die Anzahl der Anwendungsteile 11 ist nicht auf drei begrenzt ist und je nach Bedarf verringert oder erweitert werden kann.

Es ist vorgesehen, dass die intelligenten Vorrichtungen 13a, 13b und 13c jeweils mit der Steuereinheit abwechselnd und/oder gleichzeitig im AUS-Zustand ständig und im AN-Zustand bzw. Trennzustand über einen längeren Zeitraum hinweg bis die jeweiligen Energiespeicher 17a, 17b und 17c erschöpft sind, kommunizieren. Des Weiteren ist vorgesehen, dass die Anwendungsteile 11a, 11b und 11c untereinander ebenfalls kommunizieren können, um bspw. eine vorbestimmte Reihenfolge bei der Verwendung mehrerer Handstücke nacheinander sicherzustellen. Um dies gewährleisten zu können, ist eine Kommunikation in Echtzeit vorgesehen.

Es versteht sich, dass den beschriebenen Ausführungsbeispielen und nicht maßstabsgetreuen Zeichnungen lediglich beispielhafter Charakter zukommt und sich insoweit für den Fachmann Modifikationen ohne Weiteres ergeben können, ohne dass dadurch der beschreibungsgemäße Rahmen und der durch die beigefügten Ansprüche definierte Schutzumfang verlassen werden.

## Patentansprüche

1. Kommunikationsvorrichtung für ein chirurgisches Kupplungssystem, mit:
einer ersten Kupplungsvorrichtung (1) und einer zweiten Kupplungsvorrichtung (2) mit jeweils mindestens zwei mechanisch miteinander in Eingriff bringbaren elektrischen Kupplungskontakten (3, 4, 5, 6, 7, 8, 9, 10) hat, und einen Trennzustand, einen AUS-Zustand, in welchem mindestens ein elektrischer Kupplungskontakt (4, 5) der ersten Kupplungsvorrichtung (1) und der zweiten Kupplungsvorrichtung (2) außer Eingriff stehen, und einen AN-Zustand haben, in welchem der mindestens eine elektrische Kupplungskontakt (8, 9) der ersten Kupplungsvorrichtung (1) und der zweiten Kupplungsvorrichtung (2) elektrisch leitend in Kontakt stehen;
zumindest einem vorzugsweise manuell ergreifbaren Anwendungsteil (11), in welchem ein Elektromotor (12) zum Antreiben eines an das Anwendungsteil (11) angebrachten chirurgischen Werkzeugs untergebracht ist; und
einer Steuereinheit zum Regeln und/oder Steuern des Elektromotors (12);
eine in das Anwendungsteil (11) integrierte intelligente Vorrichtung (13), die dazu ausgebildet ist, eine Kommunikation zwischen dem Anwendungsteil (11) und der Steuereinheit in dem AUS-Zustand und in dem AN-Zustand aufrechtzuerhalten, und
ein Versorgungskabel, welches zwischen dem Anwendungsteil (11) und der Steuereinheit maximal drei Leitungen (14, 15, 16) hat, wobei
die Kommunikation zwischen dem Anwendungsteil (11) und der Steuereinheit (13) kabellos erfolgt, und
in dem Anwendungsteil (11) ein Energiespeicher (17) vorgesehen oder an diesem angebracht ist, der mit der intelligenten Vorrichtung (13) elektrisch verbunden ist.

2. Kommunikationsvorrichtung gemäß Anspruch 1, wobei die intelligente Vorrichtung (13) direkt in einen Signalweg zwischen einem zweiten elektrischen Kupplungskontakt (10) der zweiten Kupplungsvorrichtung (2) und dem Elektromotor (12) geschalten ist.

3. Kommunikationsvorrichtung gemäß Anspruch 1, wobei die intelligente Vorrichtung (13) über eine Entladungsschutzsicherung verfügt.

4. Kommunikationsvorrichtung gemäß einem der Ansprüche 1 bis 3, wobei die intelligente Vorrichtung (13) mehrere Eingänge (18) für Sensorsignale aufweist, wobei die intelligente Vorrichtung (13) dazu ausgebildet ist, die Sensorsignale zu empfangen und an die Steuereinheit zu senden.

5. Kommunikationsvorrichtung gemäß einem der Ansprüche 1 bis 4, wobei die intelligente Vorrichtung (13) dazu ausgebildet ist, mit der Steuereinheit zu kommunizieren und zwischen mehreren Anwendungsteilen (11) untereinander zu kommunizieren.

6. Kommunikationsvorrichtung gemäß einem der Ansprüche 1 bis 5, wobei die Spannungsversorgung indirekt, insbesondere induktiv erfolgt.

7. Kommunikationsvorrichtung gemäß Anspruch 6, wobei die intelligente Vorrichtung (13) im Zentrum einer Spule im Signalweg positioniert ist.

8. Kommunikationsvorrichtung gemäß Anspruch 6, wobei die intelligente Vorrichtung (13) mittels Harvesting aus den Leitungen (14, 15, 16) der Spannungsversorgung des Elektromotors (12) oder streuenden Magnetfeldern des Elektromotors (12) mit Energie versorgt ist.

9. Kommunikationsvorrichtung gemäß einem der Ansprüche 1 bis 8, wobei der Energiespeicher (17) dazu ausgebildet ist, in dem AUS-Zustand geladen zu werden und im AN-Zustand und/oder der Trennzustand die intelligente Vorrichtung (13) mit Energie zu versorgen.

## Claims

1. A communication device for a surgical coupling system with:
a first coupling device (1) and a second coupling device (2) each having at least two mechanically engageable electric coupling contacts (3, 4, 5, 6, 7, 8, 9, 10), and having a separated state, an OFF state in which at least one electric coupling contact (4, 5) of the first coupling device (1) and of the second coupling device (2) are disengaged, and having an ON state in which the at least one electric coupling contact (8, 9) of the first coupling device (1) and of the second coupling device (2) are in electrically conductive contact;
at least one preferably manually grippable application part (11) in which an electric motor (12) for driving a surgical tool attached to the application part (11) is housed; and
a control unit for regulating and/or controlling the electric motor (12); an intelligent device (13) incorporated in the application part (11), configured to maintain communication between the application part (11) and the control unit in the OFF state and in the ON state, and
a supply cable having a maximum of three lines (14, 15, 16) between the application part (11) and the control unit, wherein
the communication between the application part (11) and the control unit (13) is wireless, and
an energy storage device (17) is provided in or attached to the application part (11) and is electrically connected to the intelligent device (13).

2. The communication device according to claim 1, wherein the intelligent device (13) is directly connected in a signal path between a second electric coupling contact (10) of the second coupling device (2) and the electric motor (12).

3. The communication device according to claim 1, wherein the intelligent device (13) has a discharge protection fuse.

4. The communication device according to one of claims 1 to 3, wherein the intelligent device (13) has several inputs (18) for sensor signals, wherein the intelligent device (13) is configured to receive the sensor signals and to send them to the control unit.

5. The communication device according to one of claims 1 to 4, wherein the intelligent device (13) is configured to communicate with the control unit and to communicate among several application parts (11).

6. The communication device according to one of claims 1 to 5, wherein the voltage supply is indirect, in particular inductive.

7. The communication device according to claim 6, wherein the intelligent device (13) is positioned in the center of a coil in the signal path.

8. The communication device according to claim 6, wherein the intelligent device (13) is powered by means of harvesting from the lines (14, 15, 16) of the voltage supply of the electric motor (12) or scattered magnetic fields of the electric motor (12).

9. The communication device according to one of claims 1 to 8, wherein the energy storage device (17) is configured to be charged in the OFF state and to supply power to the intelligent device (13) in the ON state and/or the disconnected state.

## Revendications

1. Dispositif de communication pour un système de couplage chirurgical avec :
un premier dispositif de couplage (1) et un second dispositif de couplage (2) avec respectivement au moins deux contacts de couplage (3, 4, 5, 6, 7, 8, 9, 10) électriques pouvant être amenés en prise mécaniquement l'un avec l'autre, et un état de séparation, un état ARRÊT, dans lequel au moins un contact de couplage (4, 5) électrique du premier dispositif de couplage (1) et du second dispositif de couplage (2) est hors prise, et un état MARCHE, dans lequel le au moins un contact de couplage (8, 9) électrique du premier dispositif de couplage (1 ) et du second dispositif de couplage (2) sont en contact de manière électroconductrice ;
au moins une partie d'application (11) pouvant être saisie de préférence manuellement dans laquelle un moteur électrique (12) est logé pour l'entraînement d'un outil chirurgical monté au niveau de la partie d'application (11) ; et
un dispositif de commande pour la régulation et/ou la commande du moteur électrique (12) ;
un dispositif (13) intelligent intégré dans la partie d'application (11) qui est configuré afin de maintenir une communication entre la partie d'application (11) et le dispositif de commande dans l'état ARRÊT et dans l'état MARCHE, et
un câble d'alimentation qui présente au maximum trois lignes (14, 15, 16) entre la partie d'application (11) et le dispositif de commande, dans lequel
la communication entre la partie d'application (11) et le dispositif de commande (13) est effectuée sans câble et
un dispositif de stockage d'énergie (17) est prévu dans la partie d'application (11) ou est monté au niveau de celle-ci, dispositif qui est électriquement relié au dispositif intelligent (13).

2. Dispositif de communication selon la revendication 1, dans lequel
le dispositif intelligent (13) est monté directement dans une voie de signal entre un second contact de couplage (10) électrique du second dispositif de couplage (2) et le moteur électrique (12).

3. Dispositif de communication selon la revendication 1, dans lequel le dispositif intelligent (13) dispose d'une fixation de protection de décharge.

4. Dispositif de communication selon l'une quelconque des revendications 1 à 3, dans lequel
le dispositif intelligent (13) présente plusieurs entrées (18) pour des signaux de capteur, dans lequel le dispositif intelligent (13) est configuré afin de recevoir les signaux de capteur et des les envoyer au dispositif de commande.

5. Dispositif de communication selon l'une quelconque des revendications 1 à 4, dans lequel
le dispositif intelligent (13) est configuré afin de communiquer avec le dispositif de commande et de faire communiquer entre eux entre plusieurs parties d'application (11).

6. Dispositif de communication selon l'une quelconque des revendications 1 à 5, dans lequel
l'alimentation en tension est effectuée indirectement, en particulier par induction.

7. Dispositif de communication selon la revendication 6, dans lequel
le dispositif intelligent (13) est positionné au centre d'une bobine dans la voie de signal.

8. Dispositif de communication selon la revendication 6, dans lequel
le dispositif intelligent (13) est alimenté en énergie par récupération à partir des lignes (14, 15, 16) de l'alimentation en tension du moteur électrique (12) ou champs magnétiques diffusants du moteur électrique (12).

9. Dispositif de communication selon l'une quelconque des revendications 1 à 8, dans lequel
le dispositif de stockage d'énergie (17) est configuré afin d'être chargé dans l'état ARRÊT et d'alimenter en énergie le dispositif intelligent (13) dans l'état MARCHE et/ou l'état de séparation.
